# FASCICULE DE BREVET EUROPEEN

(11) **EP 1 806 978 B1**
(45) Date de publication et mention de la délivrance du brevet: **14.12.2011**
(21) Numéro de dépôt: 05814932.9
(22) Date de dépôt: 02.11.2005
(51) Int. Cl.: A23L 1/23, C07D 307/32, C12P 17/04

(54) **PREPARATION STEREOSELECTIVE DE GAMMA-LACTONES**
STEREOSELEKTIVE HERSTELLUNG VON GAMMA-LACTONEN
STEREOSELECTIVE PREPARATION OF GAMMA-LACTONES

(30) Priorité: 03.11.2004 FR 0411722
(43) Date de publication de la demande: 18.07.2007
(73) Titulaire: V. Mane Fils, 06620 Bar sur Loup (FR)
(72) Inventeur: ZUCCA, Joseph, F-06130 Grasse (FR); MANE, Jean, F-06130 Grasse (FR)
(74) Mandataire: Cabinet Plasseraud
(86) Numéro de dépôt international: PCT/FR2005/002730
(87) Numéro de publication internationale: WO 2006/048551

(56) Documents cités:
- EP-A- 0 258 993
- EP-A- 0 519 481
- DE-A1- 4 126 997
- US-A- 4 560 656
- US-A- 4 950 607
- US-A- 5 457 036

## Description

La présente invention a pour objet un procédé de synthèse stéréosélective de gamma-lactones, en particulier de gamma-lactones naturelles.

Les produits « naturels » sont de plus en plus prisés par le grand public, et de ce fait les industries mettant en oeuvre des composés aromatiques ou odorants portent leurs efforts dans la mise au point de substances et de préparations aromatisantes « naturelles ». Seules les substances ayant été identifiées dans la nature peuvent prétendre à ce label ; elles sont donc actuellement produites soit à partir de plantes, soit à partir de microorganismes ; ces derniers sont de plus en plus employés, des procédés biotechnologiques permettant maintenant de synthétiser des molécules naturelles à des coûts raisonnables. C'est le cas des gamma-lactones.

Les gamma-lactones sont des molécules aromatiques constitutives de l'arôme et de la saveur de nombreux produits naturels. Par exemple, la gamma-heptalactone est connue pour son arôme et son goût de noisette ou de caramel, la gamma-nonalactone a un arôme gras, crémeux, ou de noix de coco ; la gamma-décalactone et la gamma-undécalactone ont un arôme et un goût de pêche ou d'abricot.

Les gamma-lactones existent à l'état naturel, sous leurs deux formes énantiomériques (R) et (S), l'énantiomère (R) étant toutefois prédominant.

Les gamma-lactones peuvent être produites par voie synthétique, ou par biosynthèse au moyen de microorganismes. Ainsi, EP 371 568 décrit un procédé de production de gamma-lactones au moyen d'un micro-organisme acceptable pour faire des produits alimentaires tels que *Saccharomyces cerevisiae, Debaromyces hansenii* ou *Candida boidinii.*

US 5, 112, 803 indique que la gamma-octalactone, et en particulier ses isomères optiques (R) et (S) sont utiles pour former des arômes et des saveurs de beurre, et décrit un procédé pour augmenter l'arôme ou la saveur de matières consommables par ajout de quantités significatives de gamma-octalactones optiquement actives et d'un mélange de différents composés qui sont des sous-produits du procédé biologique décrit. Le procédé décrit dans US 5,112,803 indique qu'à partir d'acide caprylique, il est possible d'obtenir par biosynthèse à l'aide des souches de bactéries du genre *Syncephalastratum sp.* ou *Mortierella sp.,* les deux isomères (R) et (S) de la gamma-octalactone ; toutefois ce procédé n'est pas énantiosélectif.

L'intérêt des gamma-lactones dans l'industrie aromatique alimentaire et dans l'industrie de la parfumerie est important, et l'obtention de produits ayant des nuances organoleptiques différentes, représente un véritable enjeu industriel.

Il est connu que la chiralité des molécules volatiles peut induire des différences au niveau de la perception olfactive, et que les isomères optiques des gamma-lactones n'ont pas tous les mêmes notes organoleptiques : il y a donc un intérêt important à obtenir un isomère optique particulier de gamma-lactone, en particulier si cette obtention se fait suivant un procédé au moins aussi efficace, voire plus efficace que dans l'art antérieur et à un prix compétitif.

A ce jour, il n'existe pas, à la connaissance de la Demanderesse, de procédé stéréosélectif permettant d'obtenir directement un énantiomère de gamma-lactones naturelles. Les procédés connus conduisent à des mélanges d'énantiomères, la séparation de l'énantiomère souhaité s'effectuant généralement par chromatographie en phase gazeuse sur colonne capillaire de cyclodextrine substituée, ou après dérivatisation.

Un objet de l'invention est donc de proposer un procédé, efficace, économique et stéréosélectif, de synthèse de gamma-lactones par voie biologique.

L'invention concerne aussi bien la synthèse de (R) gamma-lactones que de (S) gamma-lactones . (R) et (S) désigne, au sens de la présente invention, la configuration du carbone asymétrique en position N°4 de la gamma-lactone.

Les gamma-lactones préférées pour être synthétisées selon le procédé de l'invention sont les C₅-C₂₀ gamma-lactones conforme à l'invention répond à la formule (I) : 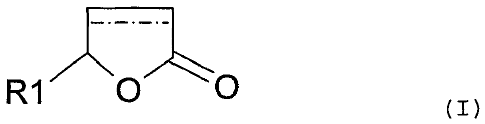 dans laquelle le cycle lactonique peut porter une insaturation entre le carbone N°2 et le carbone N°3 et dans laquelle R1 est un groupe C₁₋₁₆ alcényle, C₁₋₁₆ alcynyle ou C₁₋₁₆ alkyle, ayant éventuellement un ou plusieurs carbones substitués au delà de la position N°5. Par alcényle ou alcynyle ou alkyle substitué on entend un alcényle ou alcynyle ou alkyle dont au moins un carbone porte au moins un groupe substituant. Par groupe substituant on entend notamment un groupe hydroxyle, un groupe céto, un groupe thiol, un groupe alkyle ou un groupe alcényle.

L'invention concerne donc un procédé de préparation stéréosélective de gamma-lactone, caractérisé en ce que l'on effectue une biosynthèse par voie microbienne de gamma-lactone, en particulier de gamma-lactone de formule générale (I) ci-dessus, dans laquelle le cycle lactonique peut porter une insaturation entre le carbone N°2 et le carbone N°3, et est de préférence saturé, et dans laquelle R1 est un C₁₋₁₆ alcényle, C₁₋₁₆ alcynyle ou C₁₋₁₆ alkyle, éventuellement substitué, ladite biosynthèse étant réalisée à partir d'au moins un substrat, de préférence un acide gras, à l'aide d'une culture microbienne d'une souche choisie parmi celles genre Aspergillus sp. ou Mortierella sp., permettant une hydroxylation stéréosélective en C4 dudit substrat.

L'invention concerne la préparation de gamma-lactones par voie biologique et en particulier la biosynthèse stéréosélective de chacun des isomères optiques (R) ou (S) de gamma-lactones, à partir d'au moins un substrat, à l'aide d'une culture microbienne d'une souche appropriée.

Cette préparation comprend les étapes suivantes :
a) sélection d'une souche appropriée,
b) culture de ladite souche dans un milieu de culture approprié, ladite culture étant éventuellement précédée d'une étape de préculture de la souche,
c) ajout d'un substrat susceptible d'être transformé en gamma-lactone,
d) bioconversion du substrat en gamma-lactone,
e) récupération de la gamma-lactone produite.

Les souches microbiennes visées à l'étape a) pour la biosynthèse de la gamma-lactone selon l'invention sont celles permettant une hydroxylation spécifique du substrat spécifique en C4. Ainsi, conformément à l'invention, il est obtenu des gamma lactones dont le carbone C4 est de configuration (R) ou de configuration (S). Les souches du genre *Aspergillus sp. Mortierella sp.* appartenant à la classe 1 de microorganismes, leur utilisation ne pose pas de problème particulier tant pour la production industrielle de lactones que pour son utilisation éventuelle en alimentaire. Suivant un mode de réalisation particulier de l'invention, la souche utilisée est du genre *Aspergillus sp.,* de préférence *Aspergillus oryzae* dont on peut citer les souches de collections suivantes :

*Aspergillus oryzae* DSMZ 1861, *Aspergillus oryzae* DSMZ 1864, *Aspergillus oryzae* DSMZ 1147, *Aspergillus oryzae* DSMZ 63303, *Aspergillus oryzae* CBS 570.65, *Aspergillus oryzae* CBS 819.72, *Aspergillus oryzae* CBS 110.27, *Aspergillus oryzae* VMF 88093.

Parmi elles, *Aspergillus oryzae* DSMZ 1861 et *Aspergillus oryzae* CBS 110.27 sont préférées.

Suivant un autre mode de réalisation particulier, la souche utilisée est du genre *Mortierella sp.* dont on peut citer les espèces de collections suivantes :
*Mortierella isabellina* DSMZ 1414, *Mortierella isabellina* CBS 100559, *Mortierella isabellina* CBS 221.29, *Mortierella isabellina* CBS 194.28, *Mortierella isabellina* CBS 208.32,
*Mortierella isabellina* CBS 224.35, , *Mortierella isabellina* CBS 560.63, *Mortierella isabellina* CBS 167.80, *Mortierella isabellina* CBS 493.83, , *Mortierella isabellina* CBS 309.93,
*Mortierella isabellina* CBS 250.95, *Mortierella isabellina* CBS 109075, *Mortierella ramanniana* CBS 112.08, *Mortierella ramanniana* CBS 219.47, *Mortierella ramanniana* CBS 243.58, *Mortierella ramanniana* CBS 478.63, *Mortierella ramanniana* CBS 852.72, *Mortierella ramanniana* CBS 366.95, *Mortierella ramanniana* CBS 101226.

En effet, les inventeurs ont remarqué que, de manière surprenante et inattendue, l'utilisation d'une souche du genre *Aspergillus sp.* conduit à la production sélective de (R) gamma-lactone, et que l'utilisation d'une souche du genre *Mortierella sp.* conduit à la production sélective de (S) gamma-lactone .

Sans vouloir être lié par une quelconque théorie, il est envisageable que les conditions de culture des souches pourraient avoir une importance dans la stéréosélectivité remarquée, ainsi que dans l'aspect quantitatif de la bioconversion.

La culture visée à l'étape b) du procédé selon l'invention, comprend la réalisation d'une culture, de préférence semi-concentrée, de souches, par exemple par amplification cellulaire, dans un milieu de culture approprié. Cette culture peut être précédée par une préculture des souches dans un premier milieu de culture plus adapté aux premières étapes de multiplication de la souche.

Les conditions de culture mises en oeuvre dans le procédé stéréosélectif de l'invention doivent être telles qu'elles conduisent à la production d'un mycélium qui présente des boursouflures remplies d'inclusions (de péroxysomes en particulier). Selon le mode de réalisation préféré de l'invention, la culture cellulaire réalisée présente un mycélium « compoteux » composé de filaments cloisonnés sans conidiospores et présentant des structures renflées, remplies de ces inclusions(en particulier péroxysomes). Les conditions de culture doivent être en effet particulièrement adaptées pour éviter la sporulation du mycélium. Par ailleurs, les Inventeurs ont pu constater que l'état physiologique du mycélium, obtenu notamment du fait de la mise en oeuvre des conditions de cultures décrites dans la présente demande, (mycélium cloisonné comportant des boursouflures et renflements remplis d'inclusions, notamment de péroxysomes) pourrait avoir une influence importante sur le rendement de la réaction et permettrait d'obtenir des rendements supérieurs à ceux de l'art antérieur. L'état physiologique du mycélium pourrait également avoir une influence sur la stéréosélectivité de la réaction.

Ainsi, suivant un mode de réalisation préféré de l'invention, l'étape b) du procédé de l'invention est une étape de culture de la souche dans un milieu de culture approprié permettant l'obtention d'un mycélium cloisonné comportant des boursouflures et renflements remplies d'inclusions, notamment de péroxysomes. Le milieu de culture utilisé selon l'invention ne contient pas de peptone. Préférentiellement, le milieu de culture utilisé selon l'invention comprend du malt et/ou de l'extrait de levure. Suivant un mode de réalisation préféré, le mycélium utilisé pour l'étape c) est concentré. De préférence, la concentration du mycélium utilisé pour l'étape c) est comprise entre 5 et 15 g/l, de préférence 6 à 12 g/l, très préférentiellement 7 à 10 g/l.

Il a été particulièrement remarqué que la production de (S) gammalactone par la souche Mortierella est particulièrement favorisée, en terme de stéréosélectivité et en terme de rendement, par la mise en oeuvre d'un mycélium boursouflé et rempli d'inclusion tel que décrit ci-dessus ; en effet, l'utilisation d'un tel mycelium permettrait l'obtention d'un produit de réaction ayant un pouvoir rotatoire supérieur, en valeur absolue, à ceux de l'art antérieur ; d'autre part, le rendement obtenu par le procédé selon l'invention, et en particulier par la mise en oeuvre d'un mycélium boursouflé et rempli d'inclusions tel que décrit ci-dessus, permet l'obtention de rendements supérieurs à ceux de l'art antérieur.

L'étape c) du procédé consiste à ajouter le substrat à la culture cellulaire. Selon l'invention, la synthèse par voie biologique de gamma-lactone fait intervenir tout substrat approprié.

Par substrat approprié au sens de la présente invention, on entend les acides gras linéaires, insaturés ou non, comprenant au moins 5 carbones, et de préférence de 5 à 20 carbones, éventuellement ramifiés ou substitués au delà de la position N°5 et les esters desdits acides gras ; les esters méthyliques ou éthyliques sont préférés.

Parmi les substrats préférés, on peut citer : l'acide valérique, qui est un acide en C₅ conduisant à une gamma-valérolactone ; l'acide caproique, qui est un acide en C₆ conduisant à une gamma-hexalactone ; l'acide oenanthique qui est un acide en C₇ conduisant à une gamma-heptalactone ; l'acide caprylique qui est un acide en C₈ conduisant à une gamma-octalactone ; l'acide pélargonique qui est un acide en C₉ conduisant à une gamma-nonalactone ; l'acide décanoique qui est un acide en C₁₀ conduisant à une gamma-décalactone ; l'acide undécanoique, qui est un acide en C₁₁ conduisant à une gamma-undécalactone ; l'acide undécylénique, qui est un acide en C₁₁ conduisant à une gamma-undécénolactone ; l'acide laurique qui est un acide en C₁₂ conduisant à une gamma-dodécalactone ; l'acide myristique, qui est un acide en C₁₄ conduisant à une gamma-tetradécalactone ; l'acide palmitique , qui est un acide en C₁₆ conduisant à une gamma-hexadécalactone ; l'acide palmitoléique, qui est un acide en C16 insaturé conduisant à une gamma hexa-décénolactone, l'acide stéarique, qui est un acide en C₁₈ conduisant à une gamma-octadécalactone ; l'acide oléique, qui est un acide en C₁₈ conduisant à une gamma-octadécénolactone ; l'acide linoléique, qui est un acide en C₁₈ conduisant à une gamma-octadecadiénolactone ; l'acide linolénique, qui est un acide en C₁₈ conduisant à une gamma-octadécatriénolactone ; l'acide éicosanoique qui est un acide en C₂₀ conduisant à une gamma-éicosanolactone et leurs esters, de préférence leurs esters éthyliques ou méthyliques.

Les acides gras en C₁₃, C₁₅, C₁₇ et C₁₉ et leurs esters éthyliques ou méthyliques, bien que rares, peuvent également être oxydés et conduire respectivement aux gamma lactones en C₁₃, C₁₅, C₁₇ et C₁₉,

Il va de soi que le substrat peut être tout substrat approprié, ou un mélange de différents substrats appropriés, en particulier un mélange d'un acide déterminé et d'un ou plusieurs de ses esters.

Suivant un mode de réalisation avantageux de l'invention, le substrat est ajouté au mycélium suivant un procédé batch ou fed-batch. Suivant un mode de réalisation préféré, le substrat est ajouté en mélange avec un auxiliaire, par exemple une huile, notamment toute huile classique alimentaire telle que soja, maïs, tournesol, ou autre ou triglycérides de synthèse d'acides gras à chaînes courtes tels que miglyol, de préférence l'huile de tournesol hydrogénée ou riche en acide oléique, préalablement à sa mise en contact avec le mycélium. La présence de l'auxiliaire permet notamment de diminuer fortement l'effet corrosif ou toxique du substrat. Suivant un mode de réalisation de l'invention, la synthèse selon l'invention en utilisant la souche Mortierella isabellina s'effectue dans un milieu exempt d'huile minérale. Avantageusement, le substrat est ajouté dans des concentrations de 0.3 à 2.5g/l/h. Avantageusement, la quantité d'huile, de préférence d'huile végétale, mélangée au substrat est de 100 à 500 g/l, de préférence 150 à 300 g/l.

Une source de sucre, de préférence de glucose, est également ajoutée au milieu, en même temps que le substrat de manière à assurer la couverture des besoins énergétiques des cellules. Avantageusement, la concentration en glucose ajoutée est de 0.3 à 0.4 g/l/h

Le pH peut être régulé, suivant les besoins, pendant l'addition du substrat et pendant toute la durée de la bioconversion qui va suivre, au moyen de l'addition de toute base appropriée. Avantageusement, le pH est compris entre 4,5 et 8,5, de préférence entre 5,5 et 8 et de préférence entre 6 et 7,5.

La température est de préférence maintenue entre 27 et 30 °C, pendant la bioconversion. La durée de la bioconversion peut être de 30 à 120 heures, de préférence de 48 à 72 heures.

La bioconversion du substrat en gamma-lactone visée à l'étape d) du procédé de l'invention, est une étape de lactonisation précédée par une réaction d'hydroxylation en C4 du substrat, réalisée par la souche. Pour que cette hydroxylation puisse s'effectuer, une source d'oxygène est requise. Cette source d'oxygène est de préférence un gaz contenant de l'oxygène, très préférentiellement l'air ou l'oxygène. Le gaz est dissous en relativement grande quantité dans le milieu réactionnel.

Suivant un mode de réalisation préféré, et comme il est connu dans l'état de la technique, des agents anti-mousse, notamment des huiles siliconées ou des polymères de polyéthylène glycol estérifiés par des acides gras, sont utilisés pour contrôler la mousse susceptible de se former lors de la bioconversion.

Une fois la bioconversion, c'est-à-dire l'hydroxylation spécifique et stéréoselective en C4, suivie de la lactonisation, est effectuée, l'étape e) du procédé consiste à récupérer la gamma-lactone par extraction, l'extraction de la gamma-lactone s'effectuant par tout moyen approprié. Avantageusement, l'extraction de la gamma-lactone est effectuée par hydrodistillation, éventuellement suivie d'une estérification destinée à l'élimination ultérieure du substrat n'ayant pas réagi.

Alternativement, l'extraction de la gamma-lactone est effectuée par extraction par solvant, après acidification du milieu.

Suivant une variante de l'invention l'étape e) du procédé n'est pas réalisée, et à la place, on effectue une étape e') qui consiste à poursuivre le procédé à la fin de l'étape d) par une réduction in situ de la gamma-lactone obtenue, avant extraction. L'étape e' permet d'obtenir une gamma lactone plus saturée (R) ou (S), suivant la stéréochimie de la gamma lactone obtenue à l'étape d).

Suivant un premier mode de réalisation, la réduction peut être menée jusqu'à l'obtention d'une lactone saturée. Suivant un second mode de réalisation, la réduction peut être stoppée pour obtenir une gamma-lactone dont la chaîne latérale porte moins d'insaturations que celle issue de la bioconversion de l'étape d). Selon cet autre mode de réalisation, le procédé selon l'invention est poursuivi à l'issue de l'étape d), en stoppant la régulation pH du fermenteur, et en ajoutant une levure sèche active qui peut être une levure boulangère, une levure de vinification ou une levure de brasserie et une source de sucre, notamment de glucose, dans le réacteur. Quand le pH atteint la valeur de 5,5, il est régulé à 5,5 avec une base appropriée, par exemple de la soude NaOH. On laisse incuber, de préférence pendant un temps pendant 12 à 24 heures, puis on extrait la gamma-lactone. Suivant une autre variante, la gamma-lactone issue de l'étape d) peut être réduite par une culture fraîche d'un microorganisme réducteur ou du moins placé dans des conditions réductrices, par exemple, *Saccharomyces cerevisiae* ou *Pichia etchelsii* ou *Pichia pastoris* ou *Hansenula polymorpha* ou *Bacillus subtilis* ou *Lactobacillus brevis.*

La réduction de l'étape e') aboutit à la production de gamma-lactones plus saturées que celles issues de l'étape d). Ces gamma lactones plus saturées, obtenues selon ce mode de réalisation particulier, présentent en position 4 un carbone asymétrique de même configuration que celui de la gamma lactone moins saturée dont elle dérive, la réaction de réduction ne modifiant pas la stéréoisomérie de la molécule.

Les gamma-lactones obtenues selon le procédé de l'invention ont des propriétés odorantes et gustatives telles qu'elles peuvent être utilisées dans toutes les applications de parfumerie et d'aromatique alimentaire, en particulier pour la fabrication de parfums, de matières odorantes, de compositions cosmétiques ou alimentaires, ou comme additif alimentaire.

Au sens de la présente invention, le terme parfumerie désigne non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, anti-transpirants et désodorisants corporels, lotions et crèmes de rasage, savons, crèmes, dentifrices, bains de bouche, pommades, et produits similaires; et de produits d'entretien, tels qu'assouplissants, détergents, lessives, désodorisants d'ambiance et produits similaires.

Le terme odorant est utilisé pour désigner un composé qui exhale une odeur.

Par aromatique alimentaire, on entend toute utilisation des composés de l'invention pour l'aromatisation de toute denrée alimentaire liquide ou solide, humaine ou animale notamment des boissons, des produits laitiers, des crèmes glacées.

Les gamma-lactones, (R) ou (S) ou un mélange de (R) et (S) sont utilisables en compositions parfumantes pour contribuer à donner des notes exotiques, florales, ou fruitées. Selon les applications, on utilisera l'énantiomère (S) ou l'énantiomère (R) ou encore un mélange des 2 énantiomères dans des proportions déterminées par l'homme de l'art.

De préférence, les gamma-lactones obtenues par le procédé de l'invention selon l'invention sont utilisées dans des quantités comprises entre 0,0025% et 10 % en poids par rapport au poids total de la composition dans laquelle elles sont présentes. Elles peuvent entrer dans la composition de solides ou de liquides et notamment dans la composition de gels, crèmes, pommades et/ou sprays.

Les gamma-lactones obtenues par le procédé de l'invention selon l'invention peuvent également être utilisés dans une composition elle-même odorante, ou dans une composition dans laquelle l'agent odorant est utilisé pour masquer ou neutraliser certaines odeurs.

D'autres caractéristiques et avantages de la présente invention apparaîtront clairement à la lecture des exemples donnés ci-après qui viennent illustrer l'invention sans toutefois la limiter.

### Exemple 1, Etape a : - Sélection des souches

Toutes les souches de la collection sont d'abord ensemencées sur milieu gélosé MGY et incubées pendant 72 h à 27°C ; par la suite, ces souches sont ensemencées dans des Erlenmeyers de 1 Litre contenant 100 ml de milieu au malt 1x et incubées pendant 24 h à 27°C. Le substrat, l'acide undécylénique, est alors ajouté dans le milieu de culture (5 g/l en 10 doses) et la culture est maintenue pendant encore 48 h à 120 h à 27°C.

Après olfaction et analyses de la concentration en gamma undécénolactone dans les milieux, les souches les plus intéressantes sont retenues ; cela a été le cas pour les souches de Mortierella isabellina CBS 100559, Mortierella isabellina CBS 221.29, Aspergillus oryzae DSMZ 1861 et Aspergillus oryzae CBS 110.27 qui ont par la suite été utilisées pour les essais d'optimisation en fermenteurs.

### Exemple 1, Etape b : - Préparation des cultures cellulaires

On ensemence la souche Mortierella isabellina CBS 100559 ou Mortierella isabellina CBS 221.29 ou Aspergillus oryzae DSMZ 1861 ou Aspergillus oryzae CBS 110.27 origine = tube congelé à - 80°C) sur gélose MGY, et on incube à 27°C pendant 30 heures.

On ensemence la préculture précédente dans 5 1 de milieu au malt 1x en fermenteur de 6 1 :

| | |
|---|---|
| *Extrait de malt :* | *165 g* |
| *Extrait de levure :* | *25 g* |
| *H₂O q.s.p. :* | *5 l* |
| *pH* | *6,5* |

### Mortierella isabellina

On incube à 27°C, 500 rpm, 3,5 l/l/h d'air, pH libre, pendant 30 heures

### Aspergillus oryzae

On incube à 20°C, 500 rpm, 0,05 vvm d'air, pH libre, pendant 30 h puis à 25°C, 500 rpm, 0,05 vvm d'air, pH libre, pendant 24 heures. On doit obtenir dans les deux cas un mycélium contenant beaucoup de boursouflures grosses et pleines d'inclusions (dont des péroxysomes).

On prépare ensuite 125 l de milieu milieu au malt 1,5x dans un fermenteur de 300 l :

| | |
|---|---|
| *Extrait de malt :* | *6,188 kg* |
| *Extrait de levure :* | *0,938 kg* |
| *H₂O q.s.p. :* | *125 l* |

On stérilise le milieu 40 minutes à 121°C. Le fermenteur et ses utilités sont stériles et sous pression. La température est stable et régulée à 27°C. On chasse la pression et on maintient un débit d'air de 3,5 l/l/h, soit environ 0,6 m³/h. On raccorde stérilement la base (NaOH 10 N), l'acide (H₃PO₄ 85%) et l'antimousse et le fermenteur de 6 l qui sert d'inoculum. On ajuste la vitesse d'agitation à 325 rpm, on amorce l'antimousse, puis on ensemence l'inoculum (5 1), pH libre. On maintient la vitesse d'agitation à 325 rpm et on augmente l'aération à 2,2 m³/h (0,3 vvm). On laisse pousser pendant 24 heures de façon à avoir environ 10 g/l de poids sec de mycélium : ce mycélium doit être « compoteux » et constitué de filaments comportant de nombreux renflements et boursouflures, sans spores.

### Exemple 2, étapes c et d : Conversion de l'acide laurique par les souches de Mortierella sp.

Une fois la quantité et la qualité de mycélium atteintes, on distribue le substrat (acide laurique) dans du mygliol. On distribue parallèlement du glucose en continu au débit de 0,36 g/l/h pendant 55 h. On régule le pH à 7 pendant toute la durée de la fermentation avec NaOH 5 N. On augmente la vitesse à 900 rpm et on aère au débit de 1 vvm, soit 12 m³/h. On poursuit la conversion pendant 55 heures . On obtient un rendement de 12 g/l de Gamma dodécalactone (S). A titre comparatif, des gamma-dodécalactones sont préparées suivant l'enseignement du brevet US5457036 (Han), en utilisant les souches Mortierella décrites dans le brevet US5457036;

Lorsque le procédé Han est utilisé, les rendements obtenus sont de l'ordre de 4 à 6.5 g/l ; en comparaison, lorsqu'on utilise le procédé de l'invention mettant en oeuvre un mycélium compoteux et bourré d'inclusions, le rendement est de l'ordre de 12 à 15 g/l.

### Exemple 3, étapes c et d : Conversion de l'acide caprylique par les souches de Mortierella sp.

Une fois la quantité et la qualité de mycélium atteintes, on distribue le substrat au débit de 0,75 g/l/h pendant 6 h. On distribue parallèlement du glucose en continu au débit de 0,36 g/l/h. On régule le pH à 6,5 pendant toute la durée de la fermentation avec NaOH 5 N. On augmente la vitesse à 600 rpm et on aère au débit de 3,5 l/min. On poursuit la conversion pendant 48 à 74 heures. On obtient un rendement de 15 à 25, en général environ 19 g/l de Gamma octalactone (S).

A titre comparatif, des gamma-octalactones sont préparées suivant l'enseignement du brevet EP 519481 (Farbood), en utilisant les souches Mortierella décrites dans le brevet EP 519481 ; le pouvoir rotatoire du produit obtenu selon le procédé Farbood est de - 28 °, ce qui signifie que ce produit est un mélange (R) et (S) ayant une faible supériorité en (S) ; le pouvoir rotatoire du produit obtenu selon le procédé de l'invention est de - 42°, ce qui montre une sélectivité de la réaction pour la production de (S) gamma lactone.

Lorsque le procédé Farbood est utilisé, les rendements obtenus sont de l'ordre de 7.5 à 10 g/l ; en comparaison, lorsqu'on utilise le procédé de l'invention mettant en oeuvre un mycélium compoteux et bourré d'inclusions, le rendement est de l'ordre de 15 à 25 g/l.

### Exemple 4, étapes c et d : Conversion de l'acide caproique par les souches de Mortierella sp.

Une fois la quantité et la qualité de mycélium atteintes, on distribue le substrat au débit de 0,3 g/l/h pendant 6 h. On distribue parallèlement du glucose en continu au débit de 0,36 g/l/h. On régule le pH à 6,5 pendant toute la durée de la fermentation avec NaOH 5 N. On augmente la vitesse à 600 rpm et on aère au débit de 3,5 l/min. On poursuit la conversion pendant 48 à 74 heures. On obtient un rendement de 6 g/l de Gamma hexalactone (S).

### Exemple 5, étapes c et d : Conversion de l'acide undécylénique par les souches de Mortierella sp.

Une fois la quantité et la qualité de mycélium atteintes, on distribue l'acide undécylénique au débit de 0,3 g/l/h pendant 6 H puis au débit de 0,53 g/l/h pendant 72 h : soit un total de 40 g/l. Cet acide undécylénique est distribué en mélange avec de l'huile de tournesol hydrogénée (1/4 acide-3/4 huile) ; cette huile est donc distribuée aux débits de 0,9 g/l/h puis de 1,53 g/l/h. On distribue parallèlement du glucose en continu au débit de 0,36 g/l/h pendant 72 h. On régule le pH à 7,5 pendant toute la durée de la fermentation avec NaOH 5 N. On augmente la vitesse à 505 rpm et on aère au débit de 1 vvm, soit 12 m³/h. On poursuit la conversion pendant 72 heures.

On obtient une production de 6,5 g/l de gamma undécénolactone dont la stéréoisomérie est(S).

### Exemple 6, étapes c et d : Conversion de l'acide undécanoique par les souches de Mortierella sp.

Une fois la quantité et la qualité de mycélium atteintes, on distribue l'acide undécanoique au débit de 0,3 g/l/h pendant 6 H puis au débit de 0,53 g/l/h pendant 3,5 h, puis 0,75 g/l/h pendant 3,5 h, puis 1 g/l/h pendant 48 h. Cet acide undécanoique est distribué en mélange avec de l'huile de tournesol hydrogénée (1/4 acide-3/4 huile) ; On distribue parallèlement du glucose en continu au débit de 0,36 g/l/h pendant 24 h. On régule le pH à 7,5 pendant toute la durée de la fermentation avec NaOH 5 N. On augmente la vitesse à 505 rpm et on aère au débit de 1 vvm, soit 12 m³/h. On poursuit la conversion pendant 48 heures.

On obtient une production de 19 g/l de gamma undécalactone dont la stéréoisomérie est(S).

### Exemple 7, Etapes c et d : conversion de l'acide undécylénique par les souches d'Aspergillus sp.

Une fois la quantité et la qualité de mycélium atteintes, on distribue l'acide undécylénique au débit de 0,3 g/l/h pendant 6 H puis de 0,53 g/l/h pendant 72 h : soit un total de 40 g/l. Cet acide undécylénique est distribué en mélange avec de l'huile de tournesol hydrogénée (1/4 acide-3/4 huile). On distribue parallèlement du glucose en continu au débit de 0,36 g/l/h pendant 72 h. On régule le pH à 6,5 pendant toute la durée de la fermentation avec NaOH 5 N. On aère au débit de 0,5 vvm, soit 6 m³/h. On poursuit la conversion pendant 80 heures. On obtient une production de 0,5 g/l de gamma undécénolactone dont la stéréoisomérie est (R)

### Exemple 8, Etapes c et d : conversion de l'acide caproique par les souches d'Aspergillus sp.

Une fois la quantité et la qualité de mycélium atteintes, on distribue l'acide caproique au débit de 1,64 g/l/h pendant 24 H puis de 2 g/l/h pendant 72 h : soit un total de 183 g/l. Cet acide caproique est distribué en mélange avec de l'huile de tournesol hydrogénée (1/2 acide-1/2 huile). On distribue parallèlement du glucose en continu au débit de 0,36 g/l/h. On régule le pH à 6,5 pendant toute la durée de la fermentation avec NaOH 5 N. On aère au débit de 0,5 vvm, soit 6 m³/h. Au bout de 72 H à 96 h heures, on obtient une production de 15 g/l de gamma hexalactone dont la stéréoisomérie est (R).

### Exemple 9, Etape e : extraction - purification

On acidifie à pH 1,5 avec 3 l d'acide phosphorique à 85%. On chauffe à plus de 100 °C pendant 30 minutes pour que la lactone se présente essentiellement sous sa forme cyclisée et non pas sous sa forme ouverte d'hydroxyacide. On dose la lactone, on ajoute du solvant d'extraction (cyclohexane de préférence), on agite 1 heure à température ambiante. On centrifuge et on récupère la phase organique. On dose la lactone. On concentre le solvant et on obtient ainsi un "brut" huileux. On distille sous vide. On obtient la lactone "dérésinée" et une huile épuisée. On purifie ensuite en fractionnant la lactone sous vide. On obtient un produit pur à > 99%, qui est soit de la gamma undécénolactone (> 99% S) si on a utilisé une souche de Mortierella sp, soit de la gamma undécénolactone (> 99% R) si on a utilisé une souche d'Aspergillus sp .

La gamma undécénolactone, (R) ou (S), ou un mélange de (R) et (S), ainsi que la gamma-undécalactone (R) ou (S), ou un mélange de (R) et (S) sont utilisables en compositions parfumantes pour contribuer à donner des notes exotiques, florales ou fruitées, ce qui a conduit la Demandresse à déposer le nom de marque « Tropicalone ® » donné à la gamma undécénolactone. Selon les applications, on utilisera l'énantiomère (S) ou l'énantiomère (R) ou encore un mélange des 2 énantiomères dans des proportions déterminées par l'homme de l'art.

## Revendications

1. Procédé de préparation stéréosélective de gamma-lactones, **caractérisé en ce que** l'on effectue une biosynthèse par voie microbienne de gamma-lactones de formule générale (I) 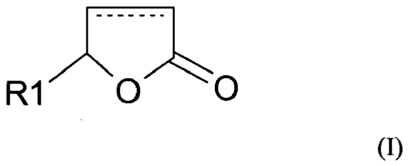 dans lesquelles le cycle lactonique porte une insaturation entre le carbone N°2 et le carbone N°3 ou est saturé, et dans lesquelles Rl est un C₁₋₁₆ alcényle, C₁₋₁₆ alcynyle ou C₁₋₁₆ alkyle, éventuellement substitué, ladite biosynthèse étant réalisée à partir d'un acide gras, à l'aide d'une culture microbienne d'une souche du genre *Aspergillus sp.* ou *Mortierella sp.,* permettant une hydroxylation stéréosélective en C4 dudit substrat, ledit procédé comprenant les étapes suivantes :
a) sélection d'une souche du genre *Aspergillus sp.* ou *Mortierella sp.,*
b) culture de ladite souche dans un milieu de culture exempt de peptones, la culture cellulaire obtenue présentant un mycélium « compoteux » composé de filaments cloisonnés sans conidiospores et présentant des structures renflées, remplies d'inclusions, ladite culture étant éventuellement précédée d'une étape de préculture de la souche,
c) ajout d'un acide gras susceptible d'être transformé en gamma-lactone,
d) bioconversion de l'acide gras en gamma-lactone de formule (I),
e) récupération de la gamma-lactone produite.

2. Procédé de préparation stéréosélective de gamma-lactones selon la revendication **1**, **caractérisé en ce que** le cycle lactonique de la gamma-lactone est saturé entre le carbone N°2 et le carbone N°3.

3. Procédé de préparation stéréosélective de (R) gamma-lactones selon la revendication **1 ou 2**, **caractérisé en ce que** la souche microbienne visée à l'étape a) est la souche *Aspergillus oryzae.*

4. Procédé de préparation stéréosélective de (S) gamma-lactones selon la revendication **1 ou 2**, **caractérisé en ce que** la souche microbienne visée à l'étape a) est la souche *Mortierella isabellina.*

5. Procédé selon l'une quelconque des revendications **1 à 4**, **caractérisé en ce que** ledit acide gras est choisi parmi les acides gras linéaires, insaturés ou non, comprenant au moins 5 carbones, et de préférence de 5 à 20 carbones, éventuellement substitués.

6. Procédé selon la revendication **5**, **caractérisé en ce que** ledit acide gras est choisi dans le groupe comprenant l'acide valérique, l'acide caproique, l'acide oenanthique, l'acide caprylique, l'acide pélargonique, l'acide décanoique, l'acide undécanoique, l'acide undécylénique, l'acide laurique, l'acide myristique, l'acide palmitique, l'acide palmitoléique, l'acide stéarique, l'acide oléique, l'acide linoléique, l'acide linolénique, l'acide éicosanoique ou un mélange de ceux-ci.

7. Procédé selon l'une quelconque des revendications **1 à 6**, **caractérisé en ce que** ledit acide gras est ajouté à l'étape c) en mélange avec au moins un auxiliaire de fabrication, de préférence choisi parmi les huiles, les triglycérides de synthèse d'acides gras à chaînes courtes, le glucose ou un mélange de ces ingrédients.

8. Procédé selon la revendication **7**, **caractérisé en ce que** l'auxiliaire est une huile choisie parmi l'huile de soja, l'huile de maïs, l'huile de tournesol, et de préférence l'huile de tournesol hydrogénée ou riche en acide oléique.

9. Procédé selon la revendication **7**, **caractérisé en ce que** l'auxiliaire est un triglycéride de synthèse d'acides gras à chaînes courtes.

10. Procédé selon la revendication **9**, **caractérisé en ce que** l'auxiliaire est le miglyol.

11. Procédé selon l'une quelconque des revendications **1 à 10**, **caractérisé en ce que** l'étape e) est une extraction par hydrodistillation de la gamma-lactone obtenue à l'issue de l'étape d), éventuellement suivie d'une estérification et élimination du substrat n'ayant pas réagi.

12. Procédé selon l'une quelconque des revendications **1 à 11**, **caractérisé en ce que** l'étape e) est une extraction par solvant de la gamma-lactone obtenue à l'issue de l'étape d).

13. Procédé selon l'une quelconque des revendications **1 à 12**, **caractérisé en ce que** l'étape e) est remplacée par une étape e') de réduction in situ de la gamma-lactone obtenue à l'issue de l'étape d).

## Claims

1. A process for the stereoselective preparation of gamma-lactones, **characterized in that** a microbial biosynthesis of gamma-lactones of general formula (I) is carried out 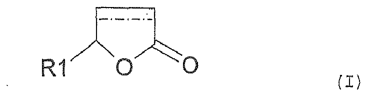 in which the lactone ring bears an unsaturation between carbon No. 2 and carbon No. 3, or is saturated, and in which R1 is a an optionally substituted C₁₋₁₆ alkenyl, C₁₋₁₆ alkynyl or C₁₋₁₆ alkyl, said biosynthesis being carried out from fatty acids using a microbial culture of the strain of genus *Aspergillus sp.* or *Mortierella sp.* that allows stereoselective hydroxylation of the substrate at C4, said process comprising the following steps:
a) selecting a strain of genus *Aspergillus sp.* or *Mortierella sp.,*
b) culturing said microbial culture in a culture medium free of peptones, the resulting cell culture having a "compot" mycelium composed of compartmentalized filaments with no conidiospores and exhibiting bulging structures, filled with inclusions, said culturing being optionally preceded by a step consisting in preculturing the microorganisms,
c) adding a fatty acid that can be converted into gamma-lactone,
d) bioconverting the fatty acid into gamma-lactone of formula (I),
e) recovering the gamma-lactone produced.

2. The stereoselective process for the preparation of gamma-lactones according to claim 1, **characterized in that** the lactone ring of the gamma-lactone is saturated between carbon No. 2 and carbon No. 3

3. The stereoselective process for the preparation of (R)-gamma-lactone according to claims 1 or 2, **characterized in that** the microbial strain targeted in step a) is the strain *Aspergillus oryzae.*

4. The stereoselective process for the preparation of (S)-gamma-lactone according to claims 1 or 2, **characterized in that** the microbial strain targeted in step a) is the strain *Mortierella isabellina.*

5. The process according to any one of claims 1 to 4, **characterized in that** said fatty acid is chosen from, optionally substituted saturated or unsaturated linear fatty acids containing at least 5 carbons, and preferably 5 to 20 carbons.

6. The process according to claim 5, **characterized in that** the fatty acid is chosen from the group comprising valeric acid, caproic acid, enanthic acid, caprylic acid, pelargonic acid, decanoic acid, undecanoic acid, undecylenic acid, lauric acid, myristic acid, palmitic acid, palmitoleic acid, stearic acid, oleic acid, linoleic acid, linolenic acid and eicosanoic acid, or a mixture thereof.

7. The process according to any one of claims 1 to 6, **characterized in that** the fatty acid is added in step c) as a mixture with at least one production auxiliary product, preferably chosen from oils, synthetic short-chain fatty acid triglycerides, glucose or a mixture of these ingredients.

8. The process according to claim 7, **characterized in that** the auxiliary product is an oil chosen from soya oil, corn oil, sunflower oil, and preferably chosen from hydrogenated or rich in oleic acid sunflower oil.

9. The process according to claim 7, **characterized in that** the auxiliary product is a synthetic short-chain fatty acid triglyceride.

10. The process according to claim 9, **characterized in that** the auxiliary product is mygliol.

11. The process according to any one of claims 1 to 10, **characterized in that** step e) is an extraction by hydrodistillation of the gamma-lactone obtained at step d), optionally followed by esterification and elimination of the substrate which has not reacted.

12. The process according to any one of claims 1 to 11, **characterized in that** step e) is a solvent extraction of the gamma-lactone obtained at the end of step d).

13. The process according to any one of claims 1 to 12, **characterized in that** step e) is replaced with a step e') consisting of the in situ reduction of the gamma-lactone obtained at the end of step d).

## Patentansprüche

1. Verfahren zur stereoselektiven Herstellung von gamma-Lactonen, **dadurch gekennzeichnet, dass** man eine mikrobielle Biosynthese von gamma-Lactonen der allgemeinen Formel (I) 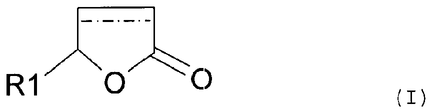 durchführt, in denen der Lactonring eine Ungesättigtheit zwischen dem Kohlenstoffatom Nr. 2 und dem Kohlenstoffatom Nr. 3 trägt oder gesättigt ist und in denen R1 ein C₁₋₁₆-Alkenyl, C₁₋₁₆-Alkinyl oder C₁₋₁₆-Alkyl ist, das gegebenenfalls substituiert ist, wobei die Biosynthese ausgehend von einer Fettsäure mithilfe einer Mikrobenkultur eines Stamms der Gattung *Aspergillus sp.* oder *Mortierella sp.* durchgeführt wird, der eine stereoselektive Hydroxylierung des Substrats an C4 gestattet, wobei das Verfahren die folgenden Schritte umfasst:
a) Auswahl eines Stamms der Gattung *Aspergillus sp.*
oder *Mortierella sp.,*
b) Kultivierung des Stamms in einem Kulturmedium ohne Peptone, wobei die erhaltene Zellkultur ein "musartiges" Mycel aufweist, das aus durch Scheidewände unterteilten Filamenten ohne Konidiosporen besteht und aufgewölbte Strukturen voller Inklusionen aufweist, wobei der Kultivierung gegebenenfalls ein Schritt der Vorkultivierung des Stamms vorausgeht,
c) Zugabe einer Fettsäure, die in gamma-Lacton umgewandelt werden kann,
d) Biokonversion der Fettsäure in gamma-Lacton der Formel (I),
e) Gewinnung des produzierten gamma-Lactons.

2. Verfahren zur stereoselektiven Herstellung von gamma-Lactonen nach Anspruch 1, **dadurch gekennzeichnet, dass** der Lactronring des gamma-Lactons zwischen dem Kohlenstoffatom Nr. 2 und dem Kohlenstoffatom Nr. 3 gesättigt ist.

3. Verfahren zur stereoselektiven Herstellung von (R)-gamma-Lactonen nach Anspruch 1 oder 2, **dadurch gekennzeichnet, dass** der im Schritt a) in Betracht gezogene Mikrobenstamm der Stamm Aspergillus oryzae ist.

4. Verfahren zur stereoselektiven Herstellung von (S)-gamma-Lactonen nach Anspruch 1 oder 2, **dadurch gekennzeichnet, dass** der im Schritt a) in Betracht gezogene Mikrobenstamm der Stamm Mortierella isabellina ist.

5. Verfahren nach einem der Ansprüche 1 bis 4, **dadurch gekennzeichnet, dass** die Fettsäure aus geraden, ungesättigten oder gesättigten, gegebenenfalls substituierten Fettsäuren ausgewählt wird, die mindestens 5 Kohlenstoffatome und vorzugsweise 5 bis 20 Kohlenstoffatome umfassen.

6. Verfahren nach Anspruch 5, **dadurch gekennzeichnet, dass** die Fettsäure aus der Gruppe ausgewählt wird, die Valeriansäure, Capronsäure, Önanthsäure, Caprylsäure, Pelargonsäure, Decansäure, Undecansäure, Undecylensäure, Laurinsäure, Myristinsäure, Palmitinsäure, Palmitoleinsäure, Stearinsäure, Ölsäure, Linolsäure, Linolensäure, Eicosansäure oder ein Gemisch davon umfasst.

7. Verfahren nach einem der Ansprüche 1 bis 6, **dadurch gekennzeichnet, dass** die Fettsäure im Schritt c) im Gemisch mit mindestens einem Herstellungshilfsstoff zugegeben wird, der vorzugsweise aus Ölen, Triglyceriden zur Synthese kurzkettiger Fettsäuren, Glucose oder einem Gemisch dieser Inhaltsstoffe ausgewählt wird.

8. Verfahren nach Anspruch 7, **dadurch gekennzeichnet, dass** der Hilfsstoff ein Öl ist, das aus Sojaöl, Maisöl, Sonnenblumenöl und vorzugsweise Sonnenblumenöl, das hydriert oder reich an Ölsäure ist, ausgewählt wird.

9. Verfahren nach Anspruch 7, **dadurch gekennzeichnet, dass** der Hilfsstoff ein Triglycerid zur Synthese kurzkettiger Fettsäuren ist.

10. Verfahren nach Anspruch 9, **dadurch gekennzeichnet, dass** der Hilfsstoff Miglyol ist.

11. Verfahren nach einem der Ansprüche 1 bis 10, **dadurch gekennzeichnet, dass** Schritt e) eine Extraktion durch Hydrodestillation des am Ende von Schritt d) erhaltenen gamma-Lactons ist, auf den gegebenenfalls eine Veresterung und Beseitigung des nicht umgesetzten Substrats folgt.

12. Verfahren nach einem der Ansprüche 1 bis 11, **dadurch gekennzeichnet, dass** Schritt e) eine Lösungsmittel-Extraktion des am Ende von Schritt d) erhaltenen gamma-Lactons ist.

13. Verfahren nach einem der Ansprüche 1 bis 12, **dadurch gekennzeichnet, dass** Schritt e) durch einen Schritt e') der in situ-Reduktion des am Ende von Schritt d) erhaltenen gamma-Lactons ersetzt wird.
